# EUROPEAN PATENT APPLICATION

(11) **EP 3 646 755 A1**
(43) Date of publication of application: **06.05.2020**
(21) Application number: 18825326.4
(22) Date of filing: 09.05.2018
(51) Int. Cl.: A45D 44/00, A61K 8/02, A61Q 1/00, G01J 3/46

(54) **SHEET, IMAGE PROCESSING DEVICE, AND IMAGE PROCESSING METHOD**

(30) Priority: 29.06.2017 JP 2017127387
(71) Applicant: Panasonic Intellectual Property Management Co., Ltd., Osaka-shi, Osaka 540-6207 (JP)
(72) Inventor: SHINODA, Masayo, Osaka-shi, Osaka 540-6207 (JP); ONODERA, Mari, Osaka-shi, Osaka 540-6207 (JP)
(74) Representative: Grünecker Patent- und Rechtsanwälte PartG mbB
(86) International application number: PCT/JP2018/017893
(87) International publication number: WO 2019/003649

(57) **Abstract**

A sheet for, when applied to a discolored part of skin, making the discolored part less conspicuous is provided. The sheet to be applied to the discolored part of the skin includes a thin film (520) having a surface applied to the skin, a first function layer (521) including a coloring material and formed on another surface of the thin film, and a second function layer (522) including a reflective material and formed on a surface of the first function layer.

## Description

### Technical Field

The present disclosure relates to a sheet appliable to the skin and an image processing apparatus and a method for processing an image used to form the sheet.

### Background Art

Techniques for making a discolored part of the skin, such as a spot on a cheek, less conspicuous currently exist (e.g., refer to PTL 1). In a technique described in PTL 1 (hereinafter referred to as an "example of the related art"), an area (hereinafter referred to as a "discolored area") corresponding to a discolored part of the skin is identified in an image obtained by capturing an image of the skin and a sheet that is appliable to the skin and on which a printing material having a color of the discolored area is printed is generated in the same size as the discolored area or a size larger than the discolored area. According to the example of the related art, a discolored part of the skin can be made less conspicuous through a simple operation for applying a sheet to the skin.

### Citation List

### Patent Literature

PTL 1: Japanese Unexamined Patent Application Publication No. 2015-43836

### Summary of Invention

In the example of the related art, however, a printing material needs to be thickly printed in order to cover a strongly discolored part, which causes a feel of heaviness when a sheet is applied. It is therefore desired to provide a technique for making a discolored part less conspicuous without causing a feel of heaviness.

An aspect of the present disclosure provides a sheet capable of making a discolored part less conspicuous without causing a feel of heaviness. Other aspects of the present disclosure provide an image processing apparatus and a method for processing an image used to generate the sheet.

The sheet according to an aspect of the present disclosure is a sheet to be applied to a discolored part of skin. The sheet includes a thin film having a surface to be applied to the skin, a first function layer including a coloring material and formed on another surface of the thin film, and a second function layer including a reflective material and formed on a surface of the first function layer.

The image processing apparatus according to another aspect of the present disclosure is an image processing apparatus used to generate the sheet. The image processing apparatus includes an image analysis unit that identifies a discolored area corresponding to the discolored part from an image obtained by capturing an image of the discolored part and a sheet information generation unit that determines thickness of the second function layer on a basis of an L* value of a Lab color space of the discolored area and generates sheet information including information regarding the determined thickness of the second function layer.

It should be noted that these general or specific aspects may be implemented as a system, a method, an integrated circuit, a computer program, a storage medium, or any selective combination thereof.

The sheet according to the present disclosure is capable of making a discolored part of the skin less conspicuous without causing a feel of heaviness. Brief Description of Drawings

[Fig. 1] Fig. 1 is a diagram illustrating an outline of a makeup assist system according to a first embodiment.
[Fig. 2] Fig. 2 is a diagram illustrating an example of the structure of a makeup sheet.
[Fig. 3A] Fig. 3A is a diagram illustrating a state in which the makeup sheet is not applied.
[Fig. 3B] Fig. 3B is a diagram illustrating a state in which the makeup sheet is applied.
[Fig. 4A] Fig. 4A is a diagram illustrating another example of the structure of the makeup sheet.
[Fig. 4B] Fig. 4B is a diagram illustrating another example of the structure of the makeup sheet.
[Fig. 4C] Fig. 4C is a diagram illustrating another example of the structure of the makeup sheet.
[Fig. 5] Fig. 5 is a block diagram illustrating an example of the configuration of an image processing apparatus according to a second embodiment.
[Fig. 6] Fig. 6 is a diagram illustrating an example of the configuration of a blocking layer information table.
[Fig. 7] Fig. 7 is a flowchart illustrating an example of an overall process performed by the image processing apparatus.
[Fig. 8] Fig. 8 is a flowchart illustrating an example of a process performed by a sheet information generation unit.
[Fig. 9] Fig. 9 is a flowchart illustrating an example of a process performed by a finish checking unit.
[Fig. 10] Fig. 10 is a diagram illustrating an example of a finish checking screen.
[Fig. 11] Fig. 11 is a block diagram illustrating an example of the hardware configuration of a computer according to the present disclosure.

### Description of Embodiments

Some embodiments of the present disclosure will be described in detail hereinafter with reference to the drawings.

### (First Embodiment)

In a first embodiment, a makeup sheet to be applied to the skin to make a discolored part such as a spot or a bruise less conspicuous will be described.

### <Outline>

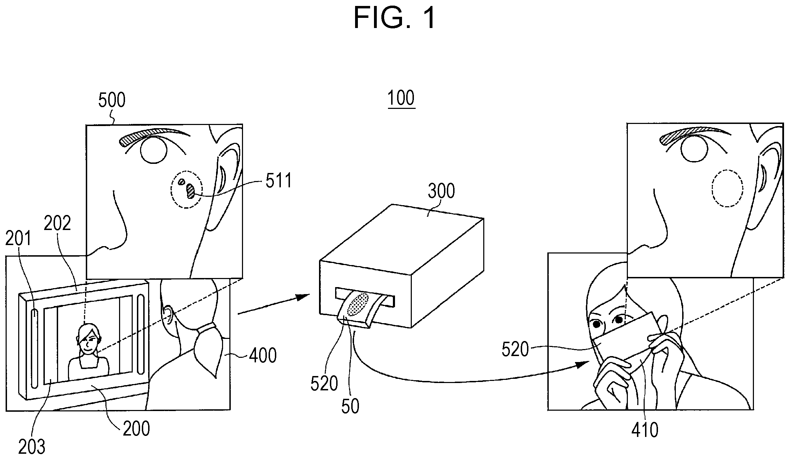

First, an outline of generation and use of a makeup sheet according to the present embodiment will be described with reference to Fig. 1.

In Fig. 1, a makeup assist system 100 includes an image processing apparatus 200 including lighting units 201, a camera 202, and a display unit 203 such as a liquid crystal display with a touch panel and a printing apparatus 300 communicably connected to the image processing apparatus 200.

The image processing apparatus 200 captures an image of a face of a user 400 located in front of the display unit 203 using the camera 202 provided near the display unit 203 with the lighting units 201 radiating visible light. The image processing apparatus 200 then generates a face image 500 by laterally reversing the captured image and displays the face image 500 on the display unit 203. As a result, the user 400 can feel as if she is looking in a mirror.

In addition, the image processing apparatus 200 identifies one or a plurality of discolored areas 511 in the face image 500 (or in the face image before the reversal). The image processing apparatus 200 then generates print data for generating a makeup sheet 50 for making a discolored part of skin 410 corresponding to the identified discolored area 511 in the face image 500 less conspicuous. The image processing apparatus 200 then transmits the print data to the printing apparatus 300 connected thereto by a certain network or cable.

The printing apparatus 300 generates the makeup sheet 50 on the basis of the print data transmitted from the image processing apparatus 200. For example, the printing apparatus 300 generates the makeup sheet 50 by stacking a coloring material and a reflective material, and the like for making a discolored part on a thin film stuck to a detachable backing member (release paper).

The user 400 can make the discolored part less conspicuous by applying the generated makeup sheet 50 to the discolored part of the skin 410.

The image processing apparatus 200 and the printing apparatus 300 are provided in, for example, a factory, a cosmetics store, a beauty salon, a medical facility, a makeup room for tidying up oneself, an event venue, or a person's house. The image processing apparatus 200 may be a portable apparatus that can be easily carried, instead.

### <Structure of Makeup Sheet>

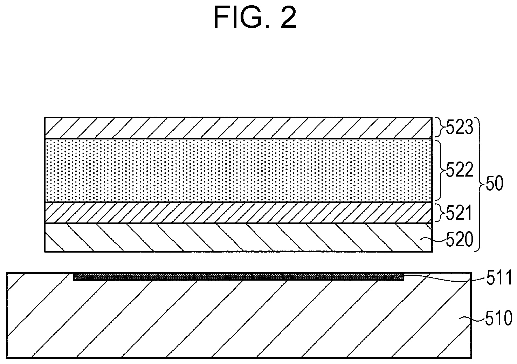

Next, the structure of the makeup sheet 50 according to the present embodiment will be described with reference to Fig. 2.

The makeup sheet 50 includes a sheet-like thin film 520, a first function layer 521 formed on the thin film 520, a second function layer 522 formed on the first function layer 521, and a third function layer 523 formed on the second function layer 522. The first function layer 521 and the second function layer 522 may be collectively referred to as a blocking layer, and the third function layer 523 may be referred to as a skin layer.

### [Thin Film]

The thin film 520 is a transparent, skin-colored, white, or translucent sheet-like member that can be comfortably applied to the human skin and that is biocompatible. The thickness of the thin film 520 may be 10 nm to 10,000 nm (10 nm to 10 µm), preferably 10 nm to 1,000 nm. When the thin film 520 is hydrophobic, the thickness of the thin film 520 is preferably 10 nm to 800 nm. The thin film 520 is rectangular when viewed in a plan. The thin film 520, however, may have any shape when viewed in a plan. For example, the thin film 520 may have a shape that matches a shape of the discolored area 511 or a shape of a part surrounding the discolored area 511.

The thin film 520 may have a shape of a sheet formed through spin coating, roll-to-roll processing, the LB technique, or the like or a shape of a fiber sheet in which fibers generated through electrospinning or the like pile up. In addition, a notch may be formed in the circumference and/or a surface of the thin film 520 so that the thin film 520 matches the shape of the discolored area 511 or the shape of the part surrounding the discolored area 511.

A material of the thin film 520 may be selected from, for example, resins such as polyglycolic acid, polylactic acid, polycaprolactone, polyethylene succinate, polyethylene terephthalate, polyethylene glycol, polypropylene glycol, nylon, polyglutamic acid, polyimide, acrylic silicone, trimethylsiloxysilicate, dimethylpolysiloxane, silicone-acrylic copolymers, alkyl acrylate-amide copolymers, polyethylene, polypropylene, and polyurethane, polymers such as polyaniline, polythiophene, polypyrrole, polyvinyl alcohol, polycarbonate, polystyrene, and Nafion, and polysaccharides such as Pullulan, cellulose (e.g., carboxymethyl cellulose, hydroxyethylcellulose, etc.), starch, chitin, chitosan, alginic acid, corn starch, pectin, arabinoxylan, glycogen, amylose, amylopectin, and hyaluronic acid. The material of the thin film 520 is preferably polylactic acid, cellulose (e.g., carboxymethyl cellulose, hydroxyethylcellulose, etc.), starch, chitin, chitosan, alginic acid, corn starch, or polyurethane.

### [First Function Layer]

The first function layer 521 is a layer formed on the thin film 520. The first function layer 521 is formed by printing a first printing material (ink) including a coloring material on the thin film 520.

The coloring material (a cosmetic material, etc.) of the first function layer 521 has a role of a control color for a discolored part. By selecting a color of a part around the discolored part (or a color similar to it) for the coloring material of the first function layer 521, therefore, the discolored part becomes less conspicuous.

The coloring material is, for example, an inorganic red pigment such as an iron oxide, a ferric hydroxide, or an iron titanate, an inorganic brown pigment such as a gamma ferric oxide, an inorganic yellow pigment such as a yellow iron oxide or loess, an inorganic black pigment such as a black iron oxide or a carbon black, an inorganic purple pigment such as manganese violet or cobalt violet, an inorganic green pigment such as a chromium hydroxide, a chromium oxide, a cobalt oxide, or a cobalt titanate, an inorganic blue pigment such as Prussian blue (iron(II) ferrocyanide) or ultramarine blue, a lake pigment obtained from one of various tar pigments, a lake pigment obtained from one of various natural pigments, a synthetic resin powder obtained by compounding some of these powders, or the like.

Particles of the first printing material may be, for example, acicular, amorphous, spherical, plate-like, or the like.

### [Second Function Layer]

The second function layer 522 is a layer formed on the first function layer 521. The second function layer 522 is formed by printing a second printing material (ink) including a reflective material on the first function layer 521.

The reflective material of the second function layer 522 has a role of increasing the brightness of a discolored part. When the brightness of a discolored part is increased, a difference in brightness between a color of the discolored part and a color of a part surrounding the discolored part becomes smaller, which makes the discolored part less conspicuous.

The reflective material is, for example, a white pigment, a pearl material, a soft focus material, a lame material, or the like. The pearl material, the soft focus material, and the lame material are, for example, titanium oxide-coated isinglass, titanium oxide-coated mica, a bismuth oxychloride, a titanium oxide-coated bismuth oxychloride, titanium oxide-coated talc, fish scale foil, titanium oxide-coated colored isinglass, non-bridging acrylic particles, polymethylsilsesquioxane, silicone, a methyl methacrylate crosspolymer, a titanium oxide, aluminum hydroxide, triethoxycaprylylsilane, kapok fiber, cellulose, polymethyl methacrylate, a methyl polymethacrylate crosspolymer, mica, an (acrylates/ethylhexyl acrylate) crosspolymer, or the like.

Particles of the second printing material may be, for example, acicular, amorphous, spherical, plate-like, or the like.

### [Third Function Layer]

The third function layer 523 is a layer formed on the second function layer 522. The third function layer 523 is formed by printing a third printing material (ink) including a coloring material on the second function layer 522.

The coloring material of the third function layer 523 has a role of coloring. In order to make a discolored part less conspicuous, a color of a part around the discolored part (or a color similar to it) may be selected for the coloring material of the third function layer 523. When the makeup sheet 50 is used as a beauty product such as a blusher, an eyeshadow, or body painting, any color may be selected for the coloring material of the third function layer 523.

The coloring material of the first function layer 521 may also be used as the coloring material of the third function layer 523. A shape of particles of the third printing material may be the same as that of the particles of the first printing material.

### <Effect Produced by Makeup Sheet>

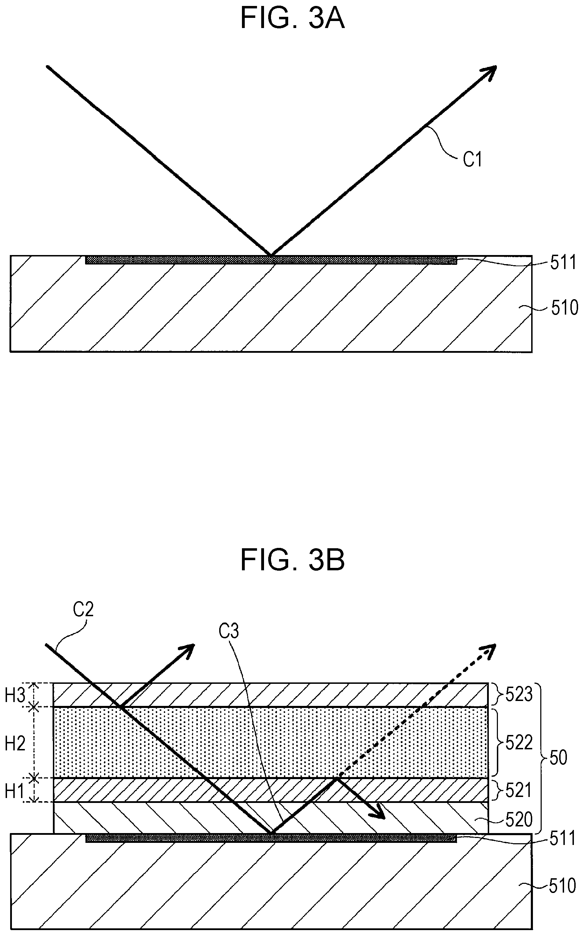

Next, an effect of making a discolored part less conspicuous produced by the makeup sheet 50 according to the present embodiment will be described with reference to Figs. 3A and 3B.

As illustrated in Fig. 3A, when the makeup sheet 50 is not applied to a discolored part, a color component included in light C1 reflected from the discolored part, that is, a color of the discolored part itself, is seen.

When the makeup sheet 50 is applied to the discolored part as illustrated in Fig. 3B, on the other hand, a color component of reflected light seen is a combination of color components of the first function layer 521, the second function layer 522, the third function layer 523, and the discolored part. Since the second function layer 522 includes the reflective material, most of incident light C2 is reflected from the second function layer 522. In addition, most of light C3 that has passed through the second function layer 522 and has been reflected from the discolored part is reflected from the second function layer 522.

In the combination of the color components of the reflected light seen, therefore, the color components of the first function layer 521 and the second function layer 522 are dominant. The percentage of the color component of the first function layer 521 is the third highest, and the percentage of the color component of the discolored part is the lowest. The makeup sheet 50 according to the present embodiment can thus make the discolored part less conspicuous.

A thickness H2 of the second function layer 522 is preferably greater than a thickness H1 of the first function layer 521. In this case, the light C3 reflected from the discolored part is sufficiently reflected from the second function layer 522, thereby making the discolored part even less conspicuous. When the thickness H2 of the second function layer 522 is too great, however, a strong feel of gloss and heaviness is undesirably caused. It is therefore preferable to provide an upper limit for the thickness H2 of the second function layer 522. The thickness of each function layer may be expressed by the number of layers achieved by printing. For example, the thickness H1 of the first function layer 521 may be expressed as one layer of printing, and the thickness H2 of the second function layer 522 may be expressed as three layers of printing.

### <Modifications of Structure of Makeup Sheet>

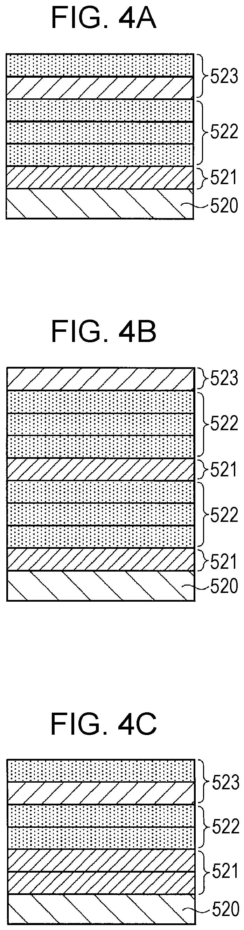

Next, modifications of the structure of the makeup sheet 50 will be described with reference to Figs. 4A, 4B, and 4C.

As illustrated in Fig. 4A, the makeup sheet 50 may further include, in the third function layer 523, a layer including a reflective material on the layer including a coloring material.

Alternatively, as illustrated in Fig. 4B, a plurality of first function layers 521 and a plurality of second function layers 522 may be alternately formed in the makeup sheet 50, and the third function layer 523 may be formed at last. This structure is appropriate when a darkly (low-brightness) discolored part is to be made less conspicuous. By increasing the brightness of the first function layers 521 as distance from a discolored part increases, for example, color gradation is caused and the discolored part is made less conspicuous in a more natural manner.

Alternatively, a plurality of second function layers 522 and a plurality of third function layers 523 may be alternately formed in the makeup sheet 50. This structure is appropriate when, for example, a discolored part is pale or when a discolored part has been sufficiently hidden but a blocking effect is to be finely adjusted. By using a color that is not largely different from a color of a part around a discolored part for the third function layers 523 and stacking the second function layers 522 and the third function layers 523, for example, the blocking effect can be finely adjusted.

In the structure in which the function layers are alternately formed, the first function layer 521 closer to the thin film 520 and the first function layer 521 located above the foregoing first function layer 521 may have the same composition but have different densities. This holds true for the second function layers 522 and the third function layers 523. When a function layer is formed through inkjet printing, the density of the function layer is a resolution (dpi), which corresponds to the density of pixels or dots. When a function layer is formed through offset printing, the density of the function layer is the density of lines. These printing methods are examples, and the function layers may be formed by any printing method.

Alternatively, as illustrated in Fig. 4C, the thickness H1 of the first function layer 521 may be two layers of printing and the thickness H2 of the second function layer 522 may be two layers of printing in the makeup sheet 50. Alternatively, although not illustrated, the thickness H3 of the third function layer 523 may be two layers of printing in the makeup sheet 50. That is, each function layer may have any thickness.

In addition, the coloring materials (printing materials) of the first function layer 521 and the second function layer 522 may be the same or different from each other.

### <Effect Produced by First Embodiment

In the makeup sheet 50 according to the present embodiment, the first function layer 521 including a coloring material is formed on the thin film 520, the second function layer 522 including a reflective material is formed on the first function layer 521, and the third function layer 523 including a coloring material is formed on the second function layer 522. When applied to a discolored part, the makeup sheet 50 does not cause a feel of heaviness and can make the discolored part sufficiently inconspicuous.

### (Second Embodiment)

In a second embodiment, the image processing apparatus 200 used to form the makeup sheet 50 described in the first embodiment will be described.

### <Configuration of Image Processing Apparatus>

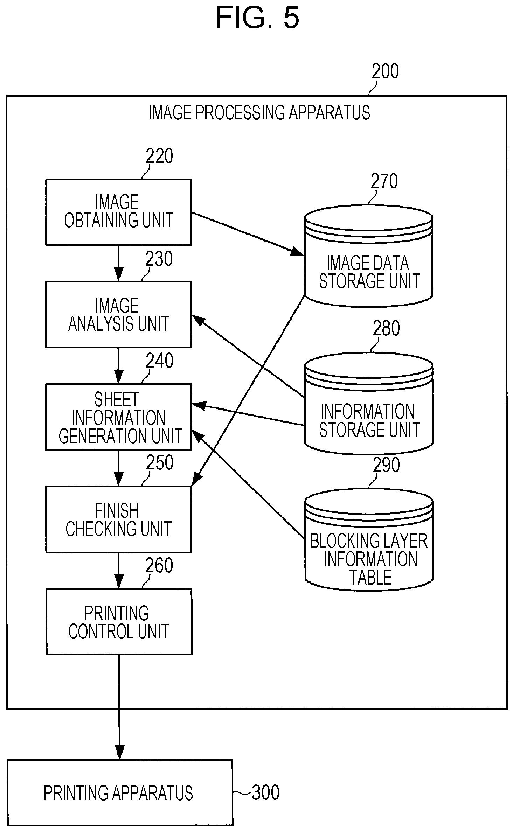

The image processing apparatus 200 will be described hereinafter with reference to Fig. 5.

The image processing apparatus 200 includes an image obtaining unit 220, an image analysis unit 230, a sheet information generation unit 240, a finish checking unit 250, a printing control unit 260, an image data storage unit 270, an information storage unit 280, and a blocking layer information table 290.

The image data storage unit 270 stores image data obtained by the image obtaining unit 220.

The information storage unit 280 stores in advance various pieces of information necessary for an image analysis conducted by the image analysis unit 230 and a determination as to sheet information made by the sheet information generation unit 240.

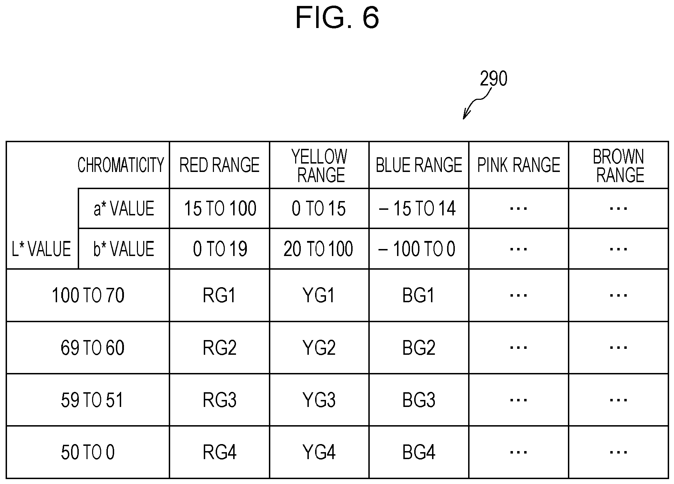

The blocking layer information table 290 manages blocking layer information suitable for the color of the discolored area 511 and the like. The blocking layer information is information for determining the structure of the blocking layer (the first function layer 521 and the second function layer 522) of the makeup sheet 50. Details of the blocking layer information table 290 will be described later (refer to Fig. 6).

The image obtaining unit 220 obtains a face image 500 of the user 400 captured by the camera 202 (refer to Fig. 1) and stores the face image 500 in the image data storage unit 270. The image obtaining unit 220 also outputs the obtained face image 500 to the image analysis unit 230.

The image analysis unit 230 analyzes the face image 500 and identifies one or a plurality of discolored areas 511 and a shape and a color of the discolored area 511. The image analysis unit 230 also identifies a color of a part around the discolored area 511. The image analysis unit 230 determines a shape of a makeup sheet 50 on the basis of the shape of the discolored area 511. Details of a process performed by the image analysis unit 230 will be described later.

The sheet information generation unit 240 determines, from the blocking layer information table 290, blocking layer information (the first function layer 521 and the second function layer 522) suitable for the color of the discolored area 511 and the like identified by the image analysis unit 230. The sheet information generation unit 240 also determines the third function layer 523 on the basis of the color of the part around the discolored area 511 identified by the image analysis unit 230. The sheet information generation unit 240 then generates sheet information including information regarding determined colors and thicknesses of the first function layer 521, the second function layer 522, and the third function layer 523 and the like. Details of a process performed by the sheet information generation unit 240 will be described later.

The finish checking unit 250 generates and displays an image at a time when the makeup sheet 50 generated on the basis of the sheet information is applied to allow the user to check a finish of the makeup sheet 50. The finish checking unit 250 also receives an instruction to adjust a color of the makeup sheet 50 from the user 400. Details of the finish checking unit 250 will be described later.

The printing control unit 260 generates print data for the printing apparatus 300 to generate the makeup sheet 50 on the basis of the sheet information generated by the sheet information generation unit 240 and adjusted by the finish checking unit 250. The printing control unit 260 then transmits the print data to the printing apparatus 300 through the certain network or cable or the like.

The printing apparatus 300 stacks the first function layer 521, the second function layer 522, and the third function layer 523 on the thin film 520 in accordance with the print data transmitted from the printing control unit 260 to generate the makeup sheet 50 according to the first embodiment.

The printing apparatus 300 may include the printing control unit 260, instead. In this case, the image processing apparatus 200 transmits the sheet information to the printing apparatus 300 through the network or the cable, and the printing control unit 260 of the printing apparatus 300 generates print data from the received sheet information. In this case, identification information for identifying the user 400 is added to the sheet information.

### <Details of Blocking Layer Information Table>

Next, details of the blocking layer information table 290 will be described with reference to Fig. 6.

The blocking layer information table 290 defines a relationship between a CIE 1976 (L*, a*, b*) color space (hereinafter referred to as a "Lab color space") and the structure of the blocking layer.

More specifically, as illustrated in Fig. 6, the blocking layer information table 290 defines grades obtained by dividing brightness (L* value) into certain ranges and color ranges obtained by dividing chromaticity (a* value and b* value) into certain ranges.

Fig. 6 illustrates an example in which brightness (L* value) is divided into grade 1 "100 to 70", grade 2 "69 to 60", grade 3 "69 to 60", and grade 4 "50 to 0" and chromaticity is divided into a red range where the a* value is "15 to 100" and the b* value is "0 to 19", a yellow range where the a* value is "0 to 15" and the b* value is "20 to 100", a blue range where the a* value is "-15 to 14" and the b* value is "-100 to 0", and the like. The color ranges may include a pink range and a brown range as well as the red, yellow, and blue ranges.

The blocking layer information table 290 associates one piece of blocking layer information with each combination of a grade and a color range. A piece of blocking layer information associated with each combination of a grade and a color range includes structure information regarding the blocking layer (the first function layer 521 and the second function layer 522) for making a discolored area 511 as inconspicuous as possible when the discolored area 511 has a brightness falling within the grade and a chromaticity falling within the color range. Here, structure information regarding the first function layer may include information regarding the thickness and color of the first function layer 521, and structure information regarding the second function layer may include information regarding the thickness and color of the second function layer 522. Information regarding thicknesses may be expressed in a unit of length, such as nanometer, or in the number of layers of printing.

As a result, the image processing apparatus 200 can generate a makeup sheet 50 in which the blocking layer (the first function layer 521 and the second function layer 522) for making a discolored area 511 less conspicuous is formed by identifying blocking layer information corresponding to the brightness (L* value) and chromaticity (a* value and b* value) of the discolored area 511 from the blocking layer information table 290.

Thresholds for the a* value and the b* value for dividing chromaticity illustrated in Fig. 6 are examples, and may be changed in accordance with a type of camera 202, a polarizing filter, a lighting environment, and/or the like. For example, chromaticity may be divided into a yellow range where (b* value) ≥ 1.284 × (a* value), a red range where (b* value) < 1.284 × (a* value), and the like.

### <Overall Process Performed by Image Processing Apparatus>

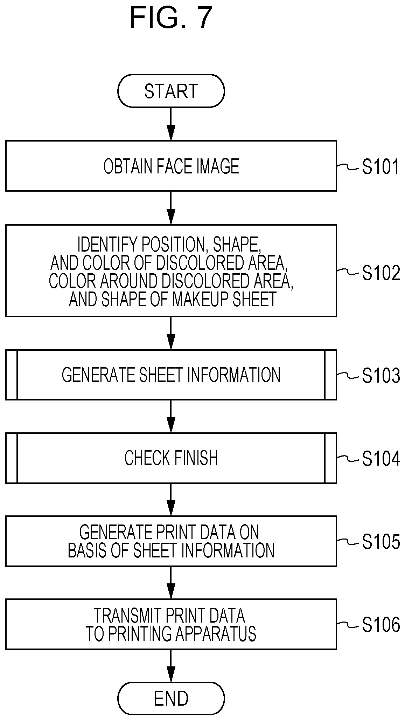

Next, a process performed by the image processing apparatus 200 will be described with reference to a flowchart of Fig. 7.

First, the image obtaining unit 220 obtains a face image 500 captured by the camera 202. The image obtaining unit 220 stores the face image 500 in the image data storage unit 270 and outputs the face image 500 to the image analysis unit 230 (S101).

Next, the image analysis unit 230 analyzes the face image 500 and identifies a position, a shape, and a color of a discolored area 511, a color of a part around the discolored area 511, and a shape of a makeup sheet 50 (S102). Details of this process performed by the image analysis unit 230 will be described later.

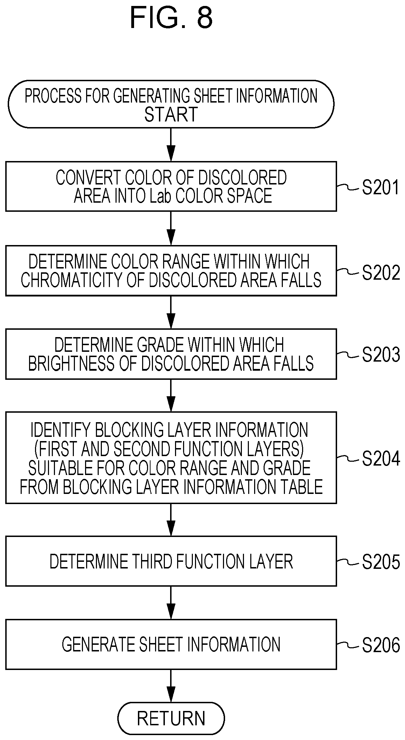

Next, the sheet information generation unit 240 generates sheet information (S103). Details of this process performed by the sheet information generation unit 240 will be described later (refer to Fig. 8).

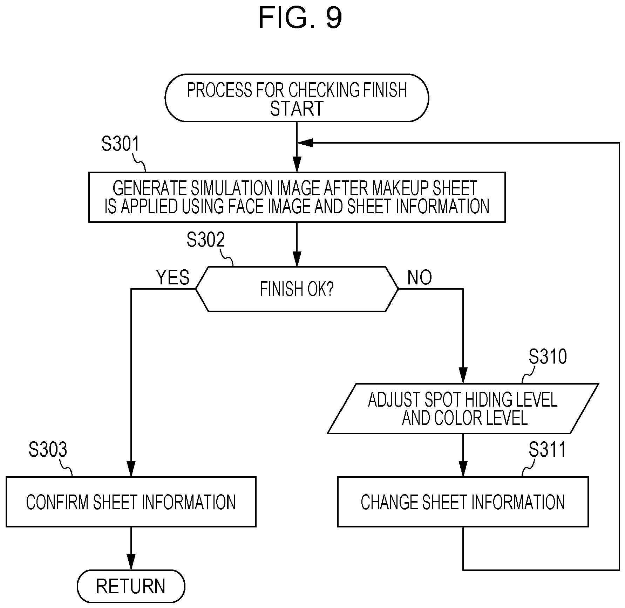

Next, the finish checking unit 250 allows the user 400 to check a finish at a time when the makeup sheet 50 generated from the sheet information is applied and receives adjustment of the sheet information from the user 400 (S104). Details of this process performed by the finish checking unit 250 will be described later (refer to Fig. 9).

Next, the printing control unit 260 generates print data on the basis of the sheet information (S105).

Lastly, the printing control unit 260 transmits the print data to the printing apparatus 300 (S106).

Upon receiving the print data, the printing apparatus 300 stacks the first function layer 521, the second function layer 522, and the third function layer 523 on the thin film 520 on the basis of the print data to generate the makeup sheet 50.

### <Details of Process Performed by Image Analysis Unit>

Next, details of the process performed by the image analysis unit 230 in step S102 will be described.

First, the image analysis unit 230 obtains an image obtained by capturing a certain color chart and determines correction values for color correction according to an image capture environment on the basis of colors of the color chart in the image.

Next, the image analysis unit 230 performs the color correction using the determined correction values and detects positions of facial parts in the face image 500 through a known process for recognizing an image, such as pattern matching.

Next, the image analysis unit 230 identifies, in an area of the face image 500 other than the facial parts, an area within a predetermined color range as a skin area.

Next, the image analysis unit 230 divides the skin area into a discolored area and a non-discolored area while using a certain pixel value (e.g., brightness) as a boundary to identify a discolored area 511. The image analysis unit 230 may treat a discolored area of a certain area or smaller as a non-discolored area.

Next, the image analysis unit 230 calculates an average of pixel values in the non-discolored area near the discolored area 511 and identifies a color of a part around the discolored area 511. The color of the part around the discolored area 511 may be determined on the basis of values measured by an external measuring device such as a spectral colorimeter or selected from color samples prepared in advance. Alternatively, the color of the part around the discolored area 511 may be determined from color information regarding a foundation, a concealer, or the like used by the user. In this case, an effect of decreasing color differences between the makeup sheet 50 and beauty products used can be expected.

Next, the image analysis unit 230 determines the shape of the makeup sheet 50 from the arrangement of the facial parts. For example, the image analysis unit 230 determines, as the shape of the makeup sheet 50, a closed shape that can cover the identified one or plurality of discolored areas 511 (or an area that extends outward from the circumference of the discolored area 511 by a certain width) while avoiding the positions of the facial parts (nostrils, eyes, mouth, eyebrows, etc.). Since a face has a three-dimensional shape and the makeup sheet 50 is basically flat, it is desirable to provide a restriction to the size of the shape of the makeup sheet 50, such as 5 cm × 5 cm or smaller.

As a result of the above process, the image analysis unit 230 identifies the position, shape, and color of the discolored area 511 in the face image 500, the color of the part around the discolored area 511, and the shape of the makeup sheet 50.

In addition, the image analysis unit 230 may identify a plurality of different discolorations in the discolored area 511. For example, the image analysis unit 230 identifies colors in the discolored area 511 in units of pixels. As a result, for example, a makeup sheet 50 for appropriately making a discolored part in which a dark part exists inside a pale part less conspicuous can be generated.

In addition, the image analysis unit 230 may convert color information regarding the discolored area 511 according to a CMYK or RGB color model into color information according to the Lab color space (hereinafter referred to as "Lab color information").

In addition, when pixels falling within a grade whose L* values constitute a smallest range (darkest grade) occupy a certain percentage or more of the discolored area 511, the image analysis unit 230 may determine the grade whose L* values are the smallest as an overall grade of the discolored area 511. As a result, a makeup sheet 50 whose angle and position need not be strictly adjusted to a discolored part when applied to the discolored part can be generated. That is, an easy-to-use makeup sheet 50 can be generated. The "certain percentage" may be managed by the information storage unit 280 as a variable parameter.

In addition, when a plurality of areas whose color ranges are different from one another exist in the discolored area 511 and the areas fall within the same grade, the image analysis unit 230 may determine a color range of a largest one of the areas as an overall color range of the discolored area 511.

In addition, the image analysis unit 230 may identify a type of discolored part from a color, a shape, and/or the like of the discolored area 511. The type of discolored part can be, for example, a pigment macule, a liver spot, a nevus spilus, a pigment cell nevus, an Ota nevus, acquired, dermal melanocytosis, erythema, purpura, vitiligo, a bruise, a mole, discoloration around pores, a tanned area, acne (pimples), an acne spot, pigmentation due to rubbing or inflammation, wrinkles, ephelides (freckles), a tattoo, a wart, a cicatrix, or the like.

### <Details of Process Performed by Sheet Information Generation Unit>

Next, details of the process performed by the sheet information generation unit 240 in step S103 will be described with reference to a flowchart of Fig. 8.

First, the sheet information generation unit 240 converts color information regarding a discolored area 511 identified by the image analysis unit 230 according to the CMYK or RGB color model into Lab color information (S201). Similarly, the sheet information generation unit 240 also converts a color of a part around the discolored area 511 into Lab color information. A process for converting the color of the part around the discolored area 511 into Lab color information may be performed by the image analysis unit 230, instead of the sheet information generation unit 240.

Next, the sheet information generation unit 240 determines a color range of the blocking layer information table 290 within which chromaticity (a* value and b* value) included in the Lab color information regarding the discolored area 511 falls (S202).

Next, the sheet information generation unit 240 determines a grade of the blocking layer information table 290 within which brightness (L* value) included in the Lab color information regarding the discolored area 511 falls (S203).

Next, the sheet information generation unit 240 identifies, from the blocking layer information table 290, blocking layer information suitable for the color range determined in step S202 and the grade determined in step S203 (S204). As a result, the structure (color, thickness, etc.) of the first function layer 521 and the second function layer 522 is determined.

Next, the sheet information generation unit 240 determines the structure of the third function layer 523 on the basis of the Lab color information regarding the part around the discolored area 511 (S205). For example, the sheet information generation unit 240 determines a color closest to the Lab color information regarding the part around the discolored area 511 as the color of the third function layer 523. The color of the third function layer 523 need not necessarily be determined on the basis of the Lab color information regarding the part around the discolored area 511. For example, the color of the third function layer 523 may be determined on the basis of Lab color information specified by the user in advance.

Lastly, the sheet information generation unit 240 generates sheet information including the structure of the first function layer 521 and the second function layer 522 determined in step S204, the structure of the third function layer 523 determined in step S205, the position and shape of the discolored area 511, the Lab color information regarding the discolored area 511, the Lab color information regarding the part around the discolored area 511, and information regarding a shape of the makeup sheet 50 (S206).

As a result of the above process, the sheet information generation unit 240 generates sheet information for generating the makeup sheet 50 described in the first embodiment.

If the image analysis unit 230 has identified a type of discolored area 511, the sheet information generation unit 240 may adjust the structure of the first function layer 521, the second function layer 522, and/or the third function layer 523 in accordance with the identified type of discolored area 511.

### <Details of Process Performed by Finish Checking Unit>

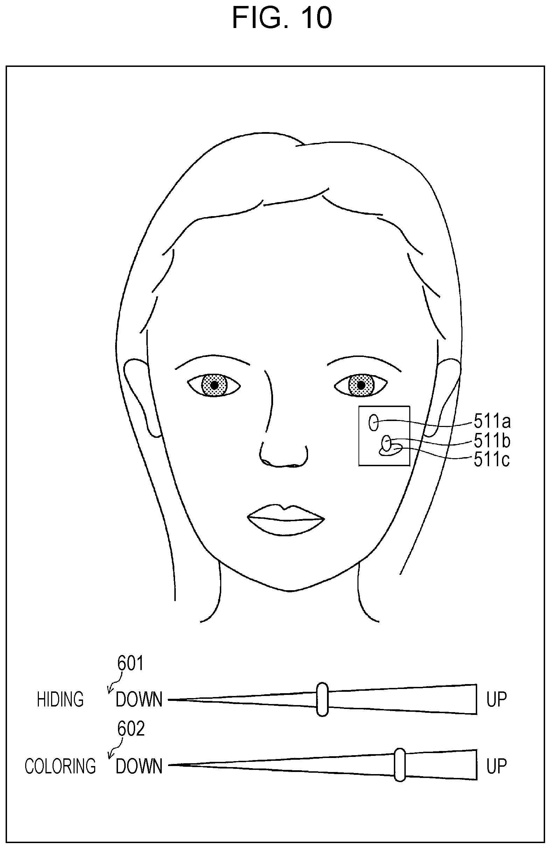

Next, details of the process performed by the finish checking unit 250 in step S104 will be described with reference to a flowchart of Fig. 9 and a finish checking screen illustrated in Fig. 10.

First, the finish checking unit 250 generates, using the face image 500 obtained in step S101 and the sheet information generated in step S105, a simulation image in which a makeup sheet 50 generated on the basis of the sheet information is applied to the face of the user 400 (S301).

Next, the finish checking unit 250 displays the simulation image generated in step S301 on the display unit 203 and asks the user 400 whether to accept a finish of the makeup sheet 50 (S302).

If the user 400 accepts the finish of the makeup sheet 50 (S302: YES), the finish checking unit 250 then confirms the sheet information (S303) and ends the process.

If the user 400 does not accept the finish of the makeup sheet 50 (S302: NO), the finish checking unit 250 then displays a UI (user interface) 601 for adjusting a hiding level of a discolored part and a UI 602 for adjusting a coloring level of the makeup sheet 50 as illustrated in Fig. 10 and receives adjustment performed by the user 400 (S310). If there are a plurality of discolored areas 511, the finish checking unit 250 may display UIs 511a, 511b, and 511c for enabling the user 400 to select a discolored area 511 to be adjusted.

The user 400 operates these Uls and adjusts the color of the makeup sheet 50 and the like. For example, the user 400 operates the UI 602 for adjusting the coloring level to make the color of the makeup sheet 50 closer to a surrounding color or match a color of a foundation usually used by the user 400. In addition, the user 400 operates the UI 601 for adjusting the hiding level of the discolored part to adjust the brightness of the makeup sheet 50.

Next, the finish checking unit 250 causes the sheet information to reflect the adjustment performed by the user in step S310 (S311). Typically, the color of the third function layer 523 reflects the adjustment of the color level based on the UI 602, and the thickness of the second function layer 522 reflects the adjustment of the hiding level of the discolored part based on the UI 601. The finish checking unit 250 then returns to step S301 and again generates a simulation image in which a makeup sheet 50 generated on the basis of the adjusted sheet information is applied.

As a result of the above process, the finish checking unit 250 allows the user 400 to check the finish of the makeup sheet 50 before proceeding to a step of printing the makeup sheet 50. In addition, the finish checking unit 250 prompts the user 400 to adjust the finish of the makeup sheet 50 as necessary.

The finish checking unit 250 is not necessarily a mandatory component of the image processing apparatus 200. A configuration in which the user is not asked to check a finish or a mode in which printing is performed without asking the user to check a finish may be employed, instead.

### <Effect Produced by Second Embodiment

The image processing apparatus 200 according to the second embodiment identifies a discolored area 511 corresponding to a discolored part in a face image obtained by capturing an image of the discolored part, determines a color of the first function layer 521 on the basis of the a* value and the b* value of the Lab color space of the identified discolored area 511, and then determines the thickness of the second function layer 522 on the basis of the L* value of the Lab color space of the discolored area 511. As a result, a makeup sheet 50 for making the discolored part less conspicuous can be generated.

### <Variations of Determination of Color of First Function Layer>

In the first and second embodiments, the color of the first function layer may be determined as in the following (1) to (3).
(1) Determination of Color Based on Color Range of Discolored Area
   If a discolored area falls within the yellow range, for example, peach orange is selected for the first function layer. This is effective in covering a pigment macule, dark circles under the eyes, or the like. If a discolored area falls within the red range, light green is selected for the first function layer. This is effective in covering an acne spot. Strength is determined in accordance with the brightness of a color of a part around the discolored area.
(2) Determination of Color Based on Color Range of Discolored Area and Color Range of Part around Discolored Area
   If the user has gotten a discolored area falling within the yellow range and the brightness (L*) of a color of a part around the discolored area is higher than a threshold, it is determined that the strength of peach orange in the first function layer is to be decreased. If the color of the part around the discolored area is bluish, pink may be added to a layer immediately above the part around the discolored area (i.e., a layer adjacent to the first function layer, which is a surface immediately above the discolored area), in order to make the user look well. As a result, the discolored area can be covered, and a part of the skin including the part around the discolored area looks healthy.
(3) Approach to Be Taken When Discolored Part Is Darkening of Skin
   In an example of an approach to be taken when a discolored part is a darkening of the skin, blue is selected for the first function layer in order to make the darkening less conspicuous and the skin look clearer using a sheet. If the user has gotten a darkening and the obtained brightness (L*) of a color of a part around a discolored area is higher than a threshold, purple is selected for the first function layer. At this time, purple may be printed over the entirety of a surface of the first function layer during forming (printing) of the first function layer, or blue and pink dots or lines may be distributed over the surface.

### <Variations of Color of Second Function Layer in Thickness Direction>

In the first and second embodiments, the same hue and the same brightness may be uniformly provided for the second function layer in a depth direction. The strength of a color determined for the second function layer may be varied in the depth direction. For example, the strength of the color may decrease in the depth direction from a side closer to the first function layer to a side closer to the third function layer.

### <Hardware Configuration>

Although the embodiments of the present disclosure have been described in detail with reference to the drawings, the above-described functions of the image processing apparatus 200 can be achieved by a computer program.

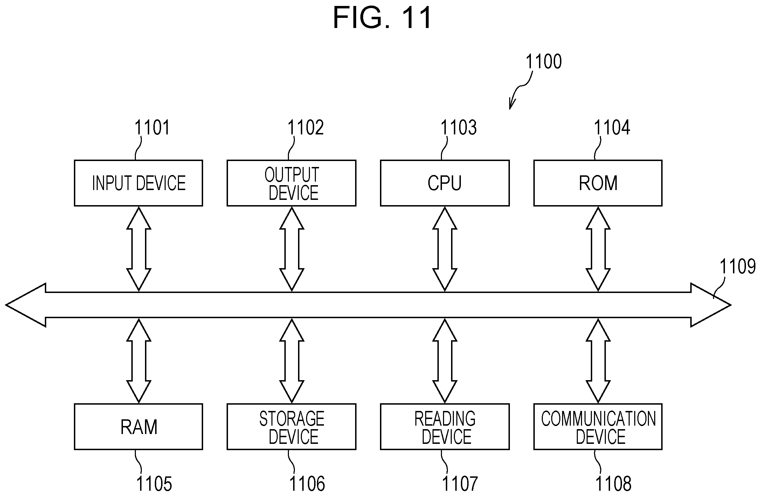

Fig. 11 is a diagram illustrating the hardware configuration of a computer that achieves the functions of the apparatus 200 using a program. A computer 1100 includes an input device 1101 such as a keyboard, a mouse, or a touch pad, an output device 1102 such as a display or a speaker, a CPU (central processing unit) 1103, a ROM (read-only memory) 1104, a RAM (random-access memory) 1105, a storage device 1106 such as a hard disk device or an SSD (solid-state device), a reading device 1107 that reads information from a storage medium such as a DVD-ROM (digital versatile disk read-only memory) or a USB (universal serial bus) memory, and a communication device 1108 that performs communication through a network. These components are connected to one another by a bus 1109.

The reading device 1107 reads the program for achieving the functions of the apparatus 200 from the storage medium storing the program and stores the program in the storage device 1106. Alternatively, the communication device 1108 communicates with a server apparatus connected to the network and stores, in the storage device 1106, the program for achieving the functions of the devices downloaded from the server apparatus.

The CPU 1103 then copies the program stored in the storage device 1106 to the RAM 1105. The CPU 1103 sequentially reads commands included in the program from the RAM 1105 and executes the commands to achieve the functions of the apparatus 200.

### Industrial Applicability

The sheet according to the present disclosure is effective especially for cosmetic purposes, and the image processing apparatus and a method for processing an image according to the present disclosure are effective in generating the sheet.

### Reference Signs List

- 100: makeup assist system
- 200: image processing apparatus
- 201: lighting unit
- 202: camera
- 203: display unit
- 220: image obtaining unit
- 230: image analysis unit
- 240: sheet information generation unit
- 250: finish checking unit
- 260: printing control unit
- 270: image data storage unit
- 280: information storage unit
- 290: blocking layer information table
- 300: printing apparatus
- 520: thin film
- 521: first function layer
- 522: second function layer
- 523: third function layer

## Claims

1. A sheet to be applied to a discolored part of skin, the sheet comprising:
a thin film having a surface to be applied to the skin;
a first function layer including a coloring material and formed on another surface of the thin film; and
a second function layer including a reflective material and formed on a surface of the first function layer.

2. The sheet according to Claim 1,
wherein thickness of the second function layer is greater than thickness of the first function layer.

3. The sheet according to Claim 1 or 2, further comprising:
a third function layer including a coloring material and formed on a surface of the second function layer.

4. The sheet according to any of Claims 1 to 3,
wherein the thin film is biocompatible and 10 nm to 10 µm in thickness.

5. An image processing apparatus used to generate the sheet according to Claim 1, the image processing apparatus comprising:
an image analysis unit that identifies a discolored area corresponding to the discolored part from an image obtained by capturing an image of the discolored part; and
a sheet information generation unit that determines thickness of the second function layer on a basis of an L* value of a Lab color space of the discolored area and generates sheet information including information regarding the determined thickness of the second function layer.

6. The image processing apparatus according to Claim 5,
wherein the sheet information generation unit determines a color of the first function layer on a basis of an a* value and a b* value of the Lab color space of the discolored area and includes information regarding the determined color of the first function layer in the sheet information.

7. The image processing apparatus according to Claim 5 or 6,
wherein the sheet further includes a third function layer including a coloring material and formed on a surface of the second function layer, and
wherein the sheet information generation unit determines a color of the third function layer on a basis of the L* value, the a* value, and the b* value of the Lab color space of a part around the discolored area and includes information regarding the determined color of the third function layer in the sheet information.

8. The image processing apparatus according to Claim 7, further comprising:
a finish checking unit that displays a simulation image in which a sheet generated on a basis of the sheet information is applied to the discolored part and receives adjustment of the color of the third function layer included in the sheet information.

9. A method for processing an image relating to generation of the sheet according to Claim 1, the method comprising:
identifying a discolored area corresponding to the discolored part in an image obtained by capturing an image of the discolored part; and
determining thickness of the second function layer on a basis of an L* value of a Lab color space of the identified discolored area and generating sheet information including information regarding the determined thickness of the second function layer.
